# EUROPEAN PATENT SPECIFICATION

(11) **EP 3 949 990 B1**
(45) Date of publication and mention of the grant of the patent: **08.07.2026**
(21) Application number: 20784328.5
(22) Date of filing: 31.03.2020
(51) Int. Cl.: A61K 39/395, A61P 1/16, A61P 35/00, A61P 43/00, C07K 16/28, A61K 47/68, C12N 15/13, A61K 31/47

(54) **MEDICAMENT FOR TREATING CANCER**
ARZNEIMITTEL ZUR BEHANDLUNG VON KREBS
MÉDICAMENT UTILISÉ DANS LE TRAITEMENT DU CANCER

(30) Priority: 01.04.2019 JP 2019070120
(43) Date of publication of application: 09.02.2022
(73) Proprietor: CHIOME BIOSCIENCE INC., Shibuya-ku Tokyo 151-0071 (JP)
(72) Inventor: NAKAMURA Koji, Tokyo 151-0071 (JP); TAKAHASHI Kota, Tokyo 151-0071 (JP); SAKAGUCHI Izumi, Tokyo 151-0071 (JP); ZHANG Lingyi, Tokyo 151-0071 (JP)
(74) Representative: J A Kemp LLP
(86) International application number: PCT/JP2020/014832
(87) International publication number: WO 2020/204033

(56) References cited:
- WO-A1-2014/054820
- WO-A1-2018/146199
- US-A1- 2009 299 038
- PERSONENI NICOLA ET AL: "Lenvatinib for the treatment of unresectable hepatocellular carcinoma: evidence to date", JOURNAL OF HEPATOCELLULAR CARCINOMA, vol. Volume 6, 1 January 2019 (2019-01-01), pages 31 - 39, XP093004122, DOI: 10.2147/JHC.S168953
- KUDO MASATOSHI: "Systemic Therapy for Hepatocellular Carcinoma: 2017 Update", vol. 93, no. Suppl. 1, 19 December 2017 (2017-12-19), CH, pages 135 - 146, XP093004038, ISSN: 0030-2414, Retrieved from the Internet <URL:https://www.karger.com/Article/Pdf/481244> DOI: 10.1159/000481244
- TSUCHIYA ATSUNORI ET AL: "Diverse perspectives to address for the future treatment of heterogeneous hepatocellular carcinoma", HELIYON, vol. 5, no. 3, 1 March 2019 (2019-03-01), GB, pages e01325, XP093004039, ISSN: 2405-8440, DOI: 10.1016/j.heliyon.2019.e01325
- KUDO, MASATOSHI ET AL.: "Lenvatinib versus sorafenib in first-line treatment of patients with unresectable hepatocellular carcinoma: a randomised phase ' non-inferiority trial", LANCET, vol. 391, 2018, pages 1163 - 73, XP085366785, DOI: 10.1016/S0140-6736(18)30207-1

## Description

### TECHNICAL FIELD

The present invention relates to a medicament and a method for treating hepatocellular carcinoma, etc.

### BACKGROUND ART

Hepatic cancer is the second leading cause of cancer-related death in the world, and about 750,000 people die of hepatic cancer a year in the world. About 780,000 patients are newly diagnosed a year, and about 80% of them are found exclusively in the Asian region including Japan and China. Hepatocellular carcinoma accounts for 85% to 90% of all hepatic cancer cases. In Japan, it is reported that the number of patients with hepatocellular carcinoma is about 42,000 and the annual number of deaths is about 26,000. Unresectable hepatocellular carcinoma is a disease with limited therapeutic options, very poor prognosis and high unmet medical needs.

As a therapeutic agent for unresectable and progressive hepatocellular carcinoma, a multikinase inhibitor against multiple receptor tyrosine kinases has been used. In more detail, sorafenib or lenvatinib has been used as a first-line drug, and regorafenib or cabozantinib has been used as a second-line drug.

Lenvatinib is one of the multikinase inhibitors, and is an orally administrable tyrosine kinase inhibitor having selective inhibitory activity against receptor-type tyrosine kinases involved in tumor angiogenesis or tumor malignancy, including VEGFR1, VEGFR2 and VEGFR3 which are members of the vascular endothelial growth factor receptor (VEGFR) family, FGFR1, FGFR2, FGFR3 and FGFR4 which are members of the fibroblast growth factor receptor (FGFR) family, as well as PDGFRα which is a member of the platelet-derived growth factor receptor (PDGFR) family, KIT, RET, etc. For the first time anywhere in the world, lenvatinib was approved in Japan in March 2018 as a primary therapeutic agent for unresectable and progressive hepatocellular carcinoma. In August 2018, lenvatinib was approved in the United States and Europe. It should be noted that lenvatinib for use a drug is generally used in the form of a mesylate salt.

As a result of an open-label, randomized phase III study (REFLECT study) for comparison between lenvatinib and sorafenib as a primary therapeutic agent for unresectable hepatocellular carcinoma, the lenvatinib group showed 40.6% efficacy, which was higher than 12.4% in the sorafenib group, and the Japanese population (of 81 patients) assigned to the lenvatinib group showed 46.9% efficacy in the REFLECT study (see Non-patent Document 1).

Thus, lenvatinib shows an efficacy as high as 40.6% against unresectable hepatocellular carcinoma, but has no effect in the remaining about 60% of the patients. Moreover, the efficacy of another primary therapeutic agent, sorafenib, is much lower, i.e., 12.4%. Thus, monotherapy with a multikinase inhibitor has a limited therapeutic effect.

### Prior Art Documents

Non-patent Document 1: Kudo M, et al., Lancet, vol. 391(10126), p. 1163-1173, 2018 Personeni Nicola ET AL: "Lenvatinib for the treatment of unresectable hepatocellular carcinoma: evidence to date",Journal of Hepatocellular Carcinoma, vol. Volume 6, 1 January 2019 (2019-01-01), pages 31-39,reports that data from the Phase III REFLECT trial showed that the multikinase inhibitor, lenvatinib, was non-inferior to sorafenib in terms of overall survival (OS). The REFLECT trial demonstrated that lenvatinib, in comparison with sorafenib, significantly increased progression-free survival, time to progression, and objective response rate for use in the treatment of unresectable hepatocellular carcinoma.

### SUMMARY OF THE INVENTION

### PROBLEM TO BE SOLVED BY THE INVENTION

Under these circumstances, there has been a demand for the development of a therapeutic agent and a therapeutic method for hepatocellular carcinoma, etc., capable of exerting a more potent and sustained antitumor effect than existing multikinase inhibitors.

### MEANS TO SOLVE THE PROBLEM

The present invention has been made in consideration of the above situation and aims to provide a pharmaceutical combination for treating hepatocellular carcinoma, etc., as shown below.
(1) A pharmaceutical combination for treating hepatocellular carcinoma, which comprises:
   lenvatinib or a prodrug thereof, or a pharmacologically acceptable salt thereof, or a hydrate or solvate thereof; and
   an antibody against human DLK-1, which has *in vivo* antitumor activity, or an antibody fragment derived from the antibody.
(2) The pharmaceutical combination according to (1) above, wherein the tumor is hepatocellular carcinoma.
(3) The pharmaceutical combination according to (1) or (2) above, wherein the antibody is a chimeric antibody or a humanized antibody.
(4) The pharmaceutical combination according to any one of (1) to (3) above, wherein the antibody is at least one selected from the group consisting of:
   (a) an antibody in which the amino acid sequences of H chain V region (heavy chain variable region) CDRs 1 to 3 are the amino acid sequences shown in SEQ ID NOs: 3 to 5, respectively, and the amino acid sequences of L chain V region (light chain variable region) CDRs 1 to 3 are the amino acid sequences shown in SEQ ID NOs: 6 to 8, respectively;
   (b) an antibody in which the amino acid sequences of H chain V region CDRs 1 to 3 are the amino acid sequences shown in SEQ ID NOs: 9 to 11, respectively, and the amino acid sequences of L chain V region CDRs 1 to 3 are the amino acid sequences shown in SEQ ID NOs: 12 to 14, respectively;
   (c) an antibody in which the amino acid sequence of the H chain V region consists of the amino acid sequence shown in SEQ ID NO: 16, and the amino acid sequence of the L chain V region consists of the amino acid sequence shown in SEQ ID NO: 18;
   (d) an antibody in which the amino acid sequence of the H chain V region consists of the amino acid sequence shown in SEQ ID NO: 20, and the amino acid sequence of the L chain V region consists of the amino acid sequence shown in SEQ ID NO: 22;
   (e) an antibody in which the amino acid sequence of the H chain V region consists of the amino acid sequence shown in SEQ ID NO: 24 or 26, and the amino acid sequence of the L chain V region consists of the amino acid sequence shown in SEQ ID NO: 28;
   (f) an antibody in which the amino acid sequence of the H chain V region consists of the amino acid sequence shown in SEQ ID NO: 30, 32, 34 or 36, and the amino acid sequence of the L chain V region consists of the amino acid sequence shown in SEQ ID NO: 46;
   (g) an antibody in which the amino acid sequence of the H chain V region consists of the amino acid sequence shown in SEQ ID NO: 38, 40, 42 or 44, and the amino acid sequence of the L chain V region consists of the amino acid sequence shown in SEQ ID NO: 46;
   (h) an antibody produced by the hybridoma of Accession No. FERM BP-10899;
   (i) an antibody produced by the hybridoma of Accession No. FERM BP-10707;
   (j) an antibody produced by the hybridoma of Accession No. FERM BP-10900; and
   (k) an antibody produced by the hybridoma of Accession No. FERM BP-11337.
(5) The pharmaceutical combination according to any one of (1) to (4) above, wherein the antibody or antibody fragment is in the form of a conjugate with a compound having antitumor activity and/or cell killing activity.
(6) The pharmaceutical combination according to any one of (1) to (5) above, which allows suppression of cancer cell proliferation or allows tumor reduction or disappearance even after completing the administration of the pharmaceutical combination.
(7) The use of:
   lenvatinib or a prodrug thereof, or a pharmacologically acceptable salt thereof, or a hydrate or solvate thereof; and
   an antibody against human DLK-1, which has *in vivo* antitumor activity, or an antibody fragment derived from the antibody for the manufacture of a therapeutic agent for hepatocellular carcinoma.
(8) A therapeutic method for hepatocellular carcinoma, which is characterized by administering a subject with:
   lenvatinib or a prodrug thereof, or a pharmacologically acceptable salt thereof, or a hydrate or solvate thereof; and
   an antibody against human DLK-1, which has *in vivo* antitumor activity, or an antibody fragment derived from the antibody.
(9) A kit for treating hepatocellular carcinoma, which comprises:
   lenvatinib or a prodrug thereof, or a pharmacologically acceptable salt thereof, or a hydrate or solvate thereof; and
   an antibody against human DLK-1, which has *in vivo* antitumor activity, or an antibody fragment derived from the antibody.

### EFFECTS OF THE INVENTION

The present invention enables the provision of a therapeutic agent and a therapeutic method, etc., capable of exerting a more potent and sustained antitumor effect than existing multikinase inhibitors in the treatment of hepatocellular carcinoma. The therapeutic agent and therapeutic method, etc., of the present invention are very useful, for example, in term of being capable of exerting an effect on patients who could not have been expected to experience any therapeutic effect.

### BRIEF DESCRIPTION OF THE DRAWINGS

Figure 1A shows the results of a measurement test for the combined effect of lenvatinib and anti-hDLK-1 antibody (HuBA-1-3D antibody) using a Hep3B xenograft model in the Example section.
Figure 1B shows the results of increases or decreases in the tumor volume of each animal (for 38 days after transplantation) in the measurement test for the combined effect of lenvatinib and anti-hDLK-1 antibody (HuBA-1-3D antibody) using the Hep3B xenograft model in the Example section.
Figure 1C shows the weights and images of the tumors excised after completion of the measurement test for the combined effect of lenvatinib and anti-hDLK-1 antibody (HuBA-1-3D antibody) using the Hep3B xenograft model in the Example section.
Figure 2A shows the results of a measurement test for the combined effect of lenvatinib and anti-hDLK-1 antibody (HuBA-1-3D antibody) using a HepG2 xenograft model in the Example section.
Figure 2B shows the tumor volumes at 34 days after tumor transplantation measured for the group receiving lenvatinib, the group receiving anti-hDLK-1 antibody and the group receiving lenvatinib and anti-hDLK-1 antibody in the measurement test for the combined effect of lenvatinib and anti-hDLK-1 antibody (HuBA-1-3D antibody) using the HepG2 xenograft model in the Example section.
Figure 3 shows the results about the sensitivity of Hep3B and HepG2 to lenvatinib, as measured by using a Hep3B xenograft model and a HepG2 xenograft model. Panel A shows the results obtained by using the Hep3B xenograft model, while panel B shows the results obtained by using the HepG2 xenograft model.

### DESCRIPTION OF EMBODIMENTS

The present invention will be further described in more detail below.

### 1. Summary of the present invention

As described above, lenvatinib is an orally administrable multikinase inhibitor having selective inhibitory activity against receptor-type tyrosine kinases involved in tumor angiogenesis or tumor malignancy, including vascular endothelial growth factor receptors (VEGFR 1 to 3) and fibroblast growth factor receptors (FGFR 1 to 4), as well as PDGFRα which is a member of the platelet-derived growth factor receptor (PDGFR) family, KIT, RET, etc. For the first time anywhere in the world, lenvatinib was approved in Japan in March 2018 as a primary therapeutic agent for unresectable and progressive hepatocellular carcinoma. In August 2018, lenvatinib was also approved in the United States and Europe. In the REFLECT study, lenvatinib showed an efficacy as high as 40.6%, but had no effect in the remaining about 60% of the patients. Thus, monotherapy with lenvatinib has a limited therapeutic effect.

To develop a therapeutic agent capable of exerting a more potent and sustained antitumor effect than existing multikinase inhibitors, the inventors of the present invention have made experiments and studies in xenograft therapeutic models using Hep3B and HepG2 cell lines derived from human hepatocellular carcinoma. As a result, the inventors of the present invention have elucidated that when lenvatinib is administered in combination with an antibody against human DLK-1 (delta-like 1 homolog (Drosophila); hereinafter also referred to as "hDLK-1"), this combined administration exerts more sustained and significantly potent tumor growth suppression and tumor reduction effects than when lenvatinib and the antibody are administered alone.

hDLK-1 is a single transmembrane type I membrane protein composed of 383 amino acid residues and is known to be expressed in adult cancers such as hepatocellular carcinoma, small cell lung cancer, pancreatic cancer, breast cancer, etc., and childhood cancers such as neuroblastoma, hepatoblastoma, rhabdomyosarcoma, virus tumor, etc. On the other hand, in normal tissues and organs, DLK-1 expression is limited to adrenal glands and pituitary glands, etc., and DLK-1 is not expressed in most organs, so that DLK-1 is suitable as a target molecule for cancer treatment. Until now, there have been developed anti-hDLK-1 monoclonal antibodies exerting tumor cell proliferation inhibition and tumor cell death induction effects including a high tumor reduction effect, and the antitumor activity of these antibodies when administered alone has been confirmed in xenograft therapeutic models using a plurality of human cancer cell lines.

According to the combination medicament and combination therapy using the combination of lenvatinib and anti-hDLK-1 antibody found by the inventors of the present invention, a sufficient therapeutic effect can be expected in patients with hepatocellular carcinoma who could not have been expected to experience a sufficient therapeutic effect when administered with multikinase inhibitors alone (i.e., who have been nonresponders). The present invention was completed in this way.

### 2. Pharmaceutical combination for cancer treatment

As described above, the pharmaceutical combination for treating hepatocellular carcinoma according to the present invention (hereinafter also referred to as "the pharmaceutical combination of the present invention") is characterized by comprising the following as active ingredients:
lenvatinib or a prodrug thereof, or a pharmacologically acceptable salt thereof, or a hydrate or solvate thereof (hereinafter also referred to herein as "lenvatinib or other form thereof"); and
anti-hDLK-1 antibody having *in vivo* antitumor activity, or an antibody fragment derived from the antibody (hereinafter also referred to herein as "anti-hDLK-1 antibody or a fragment thereof").

It should be noted that the present invention also includes: (i) a therapeutic method for hepatocellular carcinoma, which comprises using lenvatinib or other form thereof and anti-hDLK-1 antibody or a fragment thereof, more specifically, for example, administering effective amounts of lenvatinib or other form thereof and anti-hDLK-1 antibody or a fragment thereof to a subject (i.e., a patient with hepatocellular carcinoma or a patient with a risk (onset risk) of hepatocellular carcinoma, or a non-human mammal in such a state); (ii) the use of lenvatinib or other form thereof and anti-hDLK-1 antibody or a fragment thereof for the manufacture of a therapeutic agent for hepatocellular carcinoma; (iii) the use of lenvatinib or other form thereof and anti-hDLK-1 antibody or a fragment thereof for the treatment of hepatocellular carcinoma; and (iv) lenvatinib or other form thereof and anti-hDLK-1 antibody or a fragment thereof for use in the treatment of hepatocellular carcinoma.

In the present invention, the treatment of hepatocellular carcinoma includes, for example, suppression of progress, improvement of prognosis and/or prevention of recurrence in hepatocellular carcinoma.

### (1) Lenvatinib or other form thereof

Lenvatinib or other form thereof for use as an active ingredient in the pharmaceutical combination of the present invention may be any known commercially available product, but is not limited thereto, and may be synthesized, extracted and purified independently for this purpose. In the case of being synthesized, extracted and purified independently, reference may be made to the synthesis procedures described in United States Patent No. 7,612,092.

It should be noted that lenvatinib has an official name (IUPAC name) of 4-[3-chloro-4-(cyclopropylcarbamoylamino)phenoxy]-7-methoxy-quinoline-6-carboxamide, and is represented by the structural formula shown below.

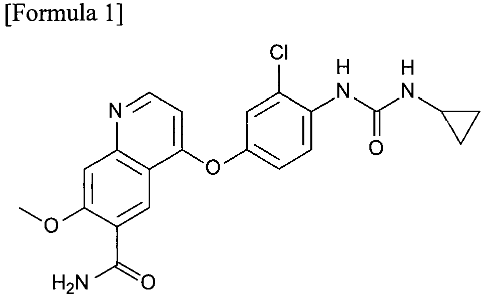

As an active ingredient in the pharmaceutical combination of the present invention, a lenvatinib derivative may also be used in combination with lenvatinib or in place of lenvatinib. Such a derivative is not limited in any way as long as it is considered to be a derivative of lenvatinib on the basis of common knowledge shared among those skilled in the art, e.g., in terms of having a chemical structure derived from lenvatinib, but preferred is a derivative having antitumor activity at the same level as lenvatinib. In the present invention, when simply referred to as lenvatinib, it is intended to mean that a derivative of lenvatinib may also be encompassed.

Examples of lenvatinib for use in the present invention include not only those which undergo *in vivo* metabolism such as oxidation, reduction, hydrolysis or conjugation, but also compounds which produce lenvatinib upon *in vivo* metabolism such as oxidation, reduction or hydrolysis (i.e., so-called prodrugs). In the present invention, such a prodrug refers to a compound prepared from its parent compound by modification with a pharmacologically acceptable group which is commonly used in prodrugs, as exemplified by a compound which is provided with properties such as improved stability and sustainability, and can be expected to exert the intended effect when converted into the parent compound in the intestinal tract or elsewhere. For example, a prodrug of lenvatinib can be prepared in a standard manner by using a prodrug-forming reagent such as a corresponding halide to introduce a prodrug-constituting group(s) as appropriate in a standard manner into any one or more groups selected from among the groups in this compound, which can be used for prodrug formation (e.g., a hydroxyl group, an amino group, other groups), optionally followed by isolation and purification. As intended here, the above prodrug-constituting groups preferably include, but are not limited to, lower alkyl-CO-, lower alkyl-O-lower alkylene-CO-, lower alkyl-OCO-lower alkylene-CO-, lower alkyl-OCO-, and lower alkyl-O-lower alkylene-OCO-, etc.

As an active ingredient in the pharmaceutical combination of the present invention, a pharmacologically acceptable salt of lenvatinib or a prodrug thereof may be used in combination with lenvatinib or a prodrug thereof or in place of lenvatinib or a prodrug thereof.

Such a pharmacologically acceptable salt is not limited in any way, but preferred examples include organic sulfonic acid salts (e.g., methanesulfonate (which is also referred to as mesylate), trifluoromethanesulfonate, ethanesulfonate, benzenesulfonate, toluenesulfonate, and camphorsulfonate), organic carboxylic acid salts (e.g., acetate, trifluoroacetate, maleate, tartrate, fumarate, and citrate), amino acid salts (e.g., aspartate, and glutamate), quaternary amine salts, alkali metal salts (e.g., sodium salt, and potassium salt), inorganic acid salts (e.g., sulfate, nitrate, perchlorate, phosphate, carbonate, and bicarbonate), halogenated hydroacid salts (e.g., hydrochloride, hydrobromide, and hydroiodide), alkaline earth metal salts (e.g., magnesium salt, and calcium salt) and so on. In one preferred embodiment, a pharmacologically acceptable salt of lenvatinib for use in the present invention may be lenvatinib mesylate.

Lenvatinib for use in the present invention encompasses all isomers possible in terms of the compound's structure (e.g., geometrical isomers, optical isomers based on asymmetric carbons, rotational isomers, stereoisomers, and tautomers) and mixtures of two or more of these isomers, and is not limited to the descriptions about the structural formula shown for convenience' sake. Moreover, lenvatinib may be in S-configuration, R-configuration or RS-configuration, and is not limited in any way. Further, lenvatinib may be present in the form of a hydrate or solvate, depending on its type. In the present invention, such a hydrate or solvate also falls within lenvatinib, and may be used as an active ingredient in the pharmaceutical combination of the present invention. Such a solvate is not limited in any way, but is exemplified by a solvate with ethanol, etc.

In the pharmaceutical combination of the present invention, the content of lenvatinib or other form thereof as an active ingredient is not limited in any way and may be determined as appropriate, but may be set to be within the range of 0.01% to 99% by weight, relative to the total weight of the pharmaceutical combination, and preferably set to be within the range of 0.01% to 30% by weight, more preferably 0.05% to 20% by weight, even more preferably 0.1% to 10% by weight, relative to the total weight of the pharmaceutical combination. The content of the active ingredient within the above range is sufficient for the pharmaceutical combination of the present invention to exert a therapeutic effect on hepatocellular carcinoma.

The pharmaceutical combination of the present invention may comprise not only lenvatinib or other form thereof, but also any other multikinase inhibitors, etc., as long as the effect of the present invention is not significantly impaired.

### (2) Anti-hDLK-1 antibody or a fragment thereof

Anti-hDLK-1 antibody (i.e., an antibody against human DLK-1, which has *in vivo* antitumor activity) for use as an active ingredient in the pharmaceutical combination of the present invention may be prepared on the basis of the following explanation.

### (i) Antigen preparation

Information on the amino acid sequence (SEQ ID NO: 2) of hDLK-1 has been made public, e.g., on the website of NCBI (GenBank) (http://www.ncbi.nlm.nih.gov/) under "Accession number: NP_003827." It should be noted that information on the nucleotide sequence (SEQ ID NO: 1) encoding the amino acid sequence of hDLK-1 has been made public on the same website under "Accession number: NM 003836."

As an antigen, it is possible to use a polypeptide or peptide (hereinafter also simply referred to as a peptide) comprising at least a part (the whole or a part) of the amino acid sequence of hDLK-1, preferably a peptide comprising at least a part (the whole or a part) of the amino acid sequence of the extracellular region (FA-1) of hDLK-1. The extracellular region of hDLK-1 refers to a region comprising six EGF-like motifs (EGF-1 to EGF-6) as described above, i.e., a region comprising amino acids at positions 24 to 244 in the amino acid sequence shown in SEQ ID NO: 2, preferably a region consisting of amino acids at positions "24" to "248 to 285" in the amino acid sequence shown in SEQ ID NO: 2 (approximately 225 to 262 amino acid residues).

As to the peptide for use as an antigen, the above "at least a part of the amino acid sequence" has no limitation on its length, and preferred is, for example, a region comprising one or two or more of the six EGF-like motifs. More preferred are, for example, a region comprising EGF-1 and EGF-2 (i.e., a region consisting of amino acids at positions 24 to 91 in the amino acid sequence shown in SEQ ID NO: 2), a region comprising EGF-3 and EGF-4 (i.e., a region consisting of amino acids at position 92 to 167 in the amino acid sequence shown in SEQ ID NO: 2), and a region comprising EGF-4, EGF-5 and EGF-6 (i.e., a region consisting of amino acids at positions 131 to 244 in the amino acid sequence shown in SEQ ID NO: 2).

The peptide for use as an antigen may be prepared either by chemical synthesis or by synthesis through genetic engineering procedures using *E. coli* or the like, and techniques well known to those skilled in the art may be used for this purpose.

For chemical synthesis, the peptide may be synthesized by well-known peptide synthesis techniques. Moreover, the synthesis may be accomplished by applying either solid phase synthesis or liquid phase synthesis. A commercially available peptide synthesizer (e.g., PSSM-8, Shimadzu Corporation, Japan) may also be used for this purpose.

For peptide synthesis through genetic engineering procedures, DNA encoding the peptide is first designed and synthesized. The design and synthesis may be accomplished, for example, by PCR techniques using a vector or the like containing the full-length hDLK-1 gene as a template and using primers which have been designed to allow synthesis of a desired DNA region. Then, the above DNA may be ligated to an appropriate vector to obtain a recombinant vector for protein expression, and this recombinant vector may be introduced into a host, such that a desired gene can be expressed therein, thereby obtaining a transformant (Molecular cloning 4th Ed. Cold Spring Harbor Laboratory Press (2012)).

As a vector, a phage or plasmid which is autonomously replicable in host microorganisms is used. Further, it is also possible to use an animal virus or insect virus vector. To prepare a recombinant vector, purified DNA may be cleaved with an appropriate restriction enzyme and ligated to a vector by being inserted into, e.g., an appropriate restriction enzyme site in the vector DNA. There is no particular limitation on the host for use in transformation as long as it is capable of expressing a desired gene. Examples include bacteria (e.g., *E. coli, Bacillus subtilis),* yeast, animal cells (e.g., COS cells, CHO cells), insect cells or insects. It is also possible to use a mammal (e.g., goat) as a host. Procedures for introduction of a recombinant vector into a host are known.

Moreover, the above transformant may be cultured, and the peptide for use as an antigen may be collected from the cultured product. The term "cultured product" is intended to mean a culture supernatant, cultured cells or cultured microorganisms, or a homogenate thereof.

After culture, when the desired peptide is produced within microorganisms or cells, the microorganisms or cells may be homogenized to thereby extract the peptide. Alternatively, when the desired peptide is produced outside microorganisms or cells, the cultured solution may be used directly or treated by centrifugation or other techniques to remove the microorganisms or cells. Then, the desired peptide may be isolated and purified by biochemical techniques commonly used for isolation and purification of peptides, as exemplified by ammonium sulfate precipitation, gel filtration, ion exchange chromatography, affinity chromatography and so on, which may be used either alone or in combination as appropriate.

In the present invention, the peptide for use as an antigen may also be obtained by *in vitro* translation using a cell-free synthesis system. In this case, it is possible to use two methods, i.e., a method in which RNA is used as a template and a method in which DNA is used as a template (transcription/translation). As a cell-free synthesis system, a commercially available system may be used, as exemplified by Expressway^{™} system (Invitrogen), PURESYSTEM^{®} (Post Genome Institute Co., Ltd., Japan), TNT system^{®} (Promega), etc.

The peptide obtained as described above may also be linked to an appropriate carrier protein such as bovine serum albumin (BSA), keyhole limpet hemocyanin (KLH), human thyroglobulin, avian gamma globulin, etc.

Moreover, the antigen may be a peptide consisting of an amino acid sequence with deletion, substitution or addition of one or more amino acids in the amino acid sequence of hDLK-1 (SEQ ID NO: 2) or its partial sequence as described above. For example, it is also possible to use a peptide consisting of an amino acid sequence with deletion of one or more (preferably one or several (e.g., 1 to 10, more preferably 1 to 5)) amino acids, with substitution of other amino acids for one or more (preferably one or several (e.g., 1 to 10, more preferably 1 to 5)) amino acids, or with addition of one or more (preferably one or several (e.g., 1 to 10, more preferably 1 to 5)) other amino acids in the amino acid sequence of hDLK-1 or its partial sequence.

In the present invention, the gene to be introduced into cells or the like may be a gene encoding a hDLK-1 protein or a partial fragment thereof or a mutated protein or fragment thereof. For example, it is possible to use a gene having the nucleotide sequence shown in SEQ ID NO: 1 or a partial sequence thereof for this purpose.

Alternatively, as a gene to be introduced into cells or the like, it is also possible to use a nucleotide sequence encoding a protein with hDLK-1 activity, or a partial sequence thereof, which is hybridizable under stringent conditions with a sequence complementary to the nucleotide sequence shown in SEQ ID NO: 1.

The term "stringent conditions" refers to washing conditions after hybridization, and is intended to mean conditions where the salt (sodium) concentration in buffer is 10 to 500 mM and the temperature is 42°C to 72°C, preferably where the above salt concentration is 50 to 300 mM and the temperature is 55°C to 68°C.

For introduction of mutations into a gene, known techniques (e.g., Kunkel method or Gapped duplex method) may be used for this purpose. For example, it is possible to use a kit for mutation introduction based on site-directed mutagenesis, as exemplified by GeneTailor^{™} Site-Directed Mutagenesis System (Invitrogen), TaKaRa Site-Directed Mutagenesis System (e.g., Prime STAR^{®} Mutagenesis Basal kit, Mutan^{®}-Super Express Km; Takara Bio Inc., Japan).

### (ii) Preparation of polyclonal antibodies

The antigen prepared above is administered to a mammal for the purpose of immunization. Such a mammal is not limited in any way, and examples include rats, mice and rabbits, with mice being particularly preferred.

The amount of the antigen to be administered per animal may be determined, as appropriate, depending on the presence or absence of an adjuvant. Examples of an adjuvant include Freund's complete adjuvant (FCA), Freund's incomplete adjuvant (FIA), aluminum hydroxide adjuvant and so on. Immunization may be primarily accomplished by injection via the intravenous, footpad, subcutaneous or intraperitoneal route, etc. Moreover, the interval between immunizations is not limited in any way, and immunization may be repeated once to 10 times, preferably twice to three times, at intervals of several days to several weeks, preferably at intervals of 1 week. Further, at 3 to 7 days after the day of the final immunization, the animals are measured for their antibody titers by enzyme immunoassay (ELISA or EIA) or radioactive immunoassay (RIA), etc., and blood may be collected at the day when each animal shows the desired antibody titer, thereby obtaining antisera. In cases where antibodies are required to be purified in the above antibody collection procedures, known techniques such as salting out with ammonium sulfate, ion exchange chromatography, gel filtration chromatography, affinity chromatography and so on may be selected as appropriate or used in combination for purification purposes. Then, polyclonal antibodies in the antisera are measured for their reactivity by ELISA assay, etc.

### (iii) Preparation of monoclonal antibody

### Collection of antibody-producing cells

The anti-hDLK-1 antibody of the present invention is not limited in any way, but is preferably a monoclonal antibody.

The antigen prepared above is administered to a mammal (e.g., rat, mouse, rabbit) for the purpose of immunization. The amount of the antigen to be administered per animal may be determined, as appropriate, depending on the presence or absence of an adjuvant. Examples of an adjuvant are the same as described above. Immunization procedures are also the same as described above. Further, at 1 to 60 days, preferably 1 to 14 days, after the day of the final immunization, antibody-producing cells are collected. Antibody-producing cells may be exemplified by spleen cells, lymph node cells and peripheral blood cells, etc., with lymph node cells or spleen cells being particularly preferred.

### Cell fusion

To obtain hybridomas (antibody-producing cell lines), cell fusion is conducted between antibody-producing cells and myeloma cells. As myeloma cells to be fused with antibody-producing cells, it is possible to use generally available established cell lines of mouse or other animal origin. Cell lines preferred for use are those having drug selectivity and having the property of not surviving in HAT selective medium (i.e., a medium containing hypoxanthine, aminopterin and thymidine) in an unfused state, but surviving only when fused with antibody-producing cells.

Examples of myeloma cells include mouse myeloma cell lines, as exemplified by P3-X63-Ag8.653, P3-X63-Ag8(X63), P3-X63-Ag8.U1(P3U1), P3/NS I/1-Ag4-1(NS1) and Sp2/0-Ag14(Sp2/0), etc. Myeloma cells may be selected as appropriate in consideration of their compatibility with antibody-producing cells.

Then, myeloma cells and antibody-producing cells are provided for cell fusion. For cell fusion, in a serum-free medium for animal cell culture (e.g., DMEM or RPMI-1640 medium), 1 × 10⁶ to 1 × 10⁷/mL of antibody-producing cells are mixed with 2 × 10⁵ to 2 × 10⁶/mL of myeloma cells. The ratio of antibody-producing cells to myeloma cells (antibody-producing cells:myeloma cells) is not limited in any way, but is usually set to preferably 1:1 to 10:1, more preferably 3:1. Then, fusion reaction is conducted in the presence of a cell fusion promoter. As a cell fusion promoter, it is possible to use polyethylene glycol having an average molecular weight of 1,000 to 6,000 daltons (D), etc. Alternatively, a commercially available cell fusion apparatus using electrical stimulation (e.g., electroporation) may also be used to cause cell fusion between antibody-producing cells and myeloma cells.

### Screening and cloning of hybridomas

After cell fusion, the cells are screened to select desired hybridomas. For screening, a cell suspension may be diluted as appropriate with, e.g., RPMI-1640 medium containing fetal bovine serum and then seeded on microtiter plates, and a selective medium may be added to each well, followed by culture while replacing the selective medium as appropriate. As a result, cells growing at around 14 days after the initiation of culture in the selective medium may be obtained as hybridomas.

Subsequently, the growing hybridomas are screened as to whether an antibody reactive to hDLK-1 is present in their culture supernatants. Screening of these hybridomas is not limited in any way and may be conducted in accordance with commonly used procedures. For example, aliquots of the culture supernatants contained in the wells where hybridomas have grown may be sampled and screened by ELISA, EIA and RIA, etc.

The fused cells may be cloned by limiting dilution or other techniques. An antibody strongly reactive to hDLK-1 is determined by flow cytometry or other techniques, and a hybridoma producing this antibody is selected and established as a clone.

### Collection of monoclonal antibody

For culture of the establish hybridoma and collection of a monoclonal antibody from the resulting cultured product, commonly used procedures, e.g., cell culture-based procedures or ascites formation procedures may be used for this purpose. The term "culture" is intended to mean that a hybridoma is allowed to grow in a culture dish or a culture bottle, or that a hybridoma is allowed to proliferate in the abdominal cavity of an animal as described below.

In cell culture-based procedures, the hybridoma may be cultured in an animal cell culture medium (e.g., 10% fetal bovine serum-containing RPMI-1640 medium, MEM medium or serum-free medium) under standard culture conditions (e.g., 37°C, 5% CO₂ concentration) for 7 to 14 days to obtain an antibody from its culture supernatant.

In the case of ascites formation procedures, the hybridoma may be intraperitoneally administered at about 1 × 10⁷ cells to an animal of the same species as the mammal from which myeloma cells are derived, whereby the hybridoma is allowed to proliferate in abundance. Then, its ascites is preferably collected after 2 to 3 weeks.

In cases where the antibody is required to be purified in the above antibody collection procedures, known techniques such as salting out with ammonium sulfate, ion exchange chromatography, gel filtration, affinity chromatography and so on may be selected as appropriate or used in combination for purification purposes.

### Selection of clone having antitumor activity

The anti-hDLK-1 antibody for use in the present invention is an antibody having *in vivo* antitumor activity.

As used herein, the term "antitumor activity" is intended to mean tumor cell (cancer cell) killing activity or tumor growth inhibitory activity. In the present invention, antitumor activity is preferably exemplified by tumor angiogenesis inhibitory activity. As to the type of human tumor (tumor cells) against which the anti-hDLK-1 antibody of the present invention can exert antitumor activity, examples include known human tumors which have been confirmed to express hDLK-1.

The presence of *in vivo* antitumor activity may be confirmed, for example, by using a cancer-bearing mouse transplanted subcutaneously with desired tumor cells, and administering this mouse with the antibody obtained as described above. In this case, the antibody may be administered either immediately after transplantation of tumor cells (Prevention model) or after confirming that the tumor has grown to a certain volume after transplantation (Treatment model). The antibody may be administered in any manner, for example, may be administered once every 3 days at a dose of 20 mg/kg body weight via the intraperitoneal route. In the case of the Prevention model, the presence or absence of antitumor activity and the level thereof may be evaluated on the basis of tumorigenesis frequency and tumor volume. In the case of the Treatment model, the presence or absence of antitumor activity and the level thereof may be evaluated on the basis of tumor volume and tumor weight.

In the present invention, the anti-hDLK-1 antibody having *in vivo* antitumor activity is not limited in any way, but it is possible to use anti-hDLK-1 antibodies as disclosed in the following gazettes: WO2008/056833, WO2009/116670 and WO2014/054820.

In more detail, examples include anti-hDLK-1 antibodies (a) to (k) shown below, and at least one of them may be used:
(a) an antibody in which the amino acid sequences of H chain V region CDRs 1 to 3 are the amino acid sequences shown in SEQ ID NOs: 3 to 5, respectively, and the amino acid sequences of L chain V region CDRs 1 to 3 are the amino acid sequences shown in SEQ ID NOs: 6 to 8, respectively;
(b) an antibody in which the amino acid sequences of H chain V region CDRs 1 to 3 are the amino acid sequences shown in SEQ ID NOs: 9 to 11, respectively, and the amino acid sequences of L chain V region CDRs 1 to 3 are the amino acid sequences shown in SEQ ID NOs: 12 to 14, respectively;
(c) an antibody in which the amino acid sequence of the H chain V region consists of the amino acid sequence shown in SEQ ID NO: 16 (the corresponding nucleotide sequence is SEQ ID NO: 15), and the amino acid sequence of the L chain V region consists of the amino acid sequence shown in SEQ ID NO: 18 (the corresponding nucleotide sequence is SEQ ID NO: 17), and it should be noted that CDR sequences in the H chain V region and L chain V region of this antibody are the same as those in the antibody shown in (a) above;
(d) an antibody in which the amino acid sequence of the H chain V region consists of the amino acid sequence shown in SEQ ID NO: 20 (the corresponding nucleotide sequence is SEQ ID NO: 19), and the amino acid sequence of the L chain V region consists of the amino acid sequence shown in SEQ ID NO: 22 (the corresponding nucleotide sequence is SEQ ID NO: 21), and it should be noted that CDR sequences in the H chain V region and L chain V region of this antibody are the same as those in the antibody shown in (b) above;
(e) an antibody (humanized antibody) in which the amino acid sequence of the H chain V region consists of the amino acid sequence shown in SEQ ID NO: 24 or 26 (the corresponding nucleotide sequence is SEQ ID NO: 23 or 25), and the amino acid sequence of the L chain V region consists of the amino acid sequence shown in SEQ ID NO: 28 (the corresponding nucleotide sequence is SEQ ID NO: 27), and it should be noted that CDR sequences in the H chain V region and L chain V region of this antibody are the same as those in the antibody shown in (a) above;
(f) an antibody (humanized antibody) in which the amino acid sequence of the H chain V region consists of the amino acid sequence shown in SEQ ID NO: 30, 32, 34 or 36 (the corresponding nucleotide sequence is SEQ ID NO: 29, 31, 33 or 35), and the amino acid sequence of the L chain V region consists of the amino acid sequence shown in SEQ ID NO: 46 (the corresponding nucleotide sequence is SEQ ID NO: 45), and it should be noted that CDR sequences in the H chain V region and L chain V region of this antibody are the same as those in the antibody shown in (b) above;
(g) an antibody (humanized antibody) in which the amino acid sequence of the H chain V region consists of the amino acid sequence shown in SEQ ID NO: 38, 40, 42 or 44 (the corresponding nucleotide sequence is SEQ ID NO: 37, 39, 41 or 43), and the amino acid sequence of the L chain V region consists of the amino acid sequence shown in SEQ ID NO: 46 (the corresponding nucleotide sequence is SEQ ID NO: 45), and it should be noted that CDR sequences in the H chain V region and L chain V region of this antibody are the same as those in the antibody shown in (b) above;
(h) an antibody produced by the hybridoma of Accession No. FERM BP-10899;
(i) an antibody produced by the hybridoma of Accession No. FERM BP-10707;
(j) an antibody produced by the hybridoma of Accession No. FERM BP-10900; and
(k) an antibody produced by the hybridoma of Accession No. FERM BP-11337.

With regard to the antibody shown in (f) above, the amino acid sequence shown in SEQ ID NO: 32 comprises a substitution from alanine (A) to glycine (G) at position 24 in the amino acid sequence shown in SEQ ID NO: 30,
the amino acid sequence shown in SEQ ID NO: 34 comprises a substitution from threonine (T) to lysine (K) at position 74 in the amino acid sequence shown in SEQ ID NO: 30, and
the amino acid sequence shown in SEQ ID NO: 36 comprises a substitution from alanine (A) to glycine (G) at position 24 and a substitution from threonine (T) to lysine (K) at position 74 in the amino acid sequence shown in SEQ ID NO: 30.

Likewise, with regard to the antibody shown in (g) above, the amino acid sequence shown in SEQ ID NO: 40 comprises a substitution from alanine (A) to glycine (G) at position 24 in the amino acid sequence shown in SEQ ID NO: 38,
the amino acid sequence shown in SEQ ID NO: 42 comprises a substitution from threonine (T) to lysine (K) at position 74 in the amino acid sequence shown in SEQ ID NO: 38, and
the amino acid sequence shown in SEQ ID NO: 44 comprises a substitution from alanine (A) to glycine (G) at position 24 and a substitution from threonine (T) to lysine (K) at position 74 in the amino acid sequence shown in SEQ ID NO: 38.

Such modified antibodies comprising amino acid substitutions in the antibodies (humanized antibodies) shown in (f) and (g) above are antibodies with much higher avidity (antigen binding activity), for example, such that they are capable of retaining their binding activity to cancer cells showing low antigen expression levels on the cell surface. In addition, these modified antibodies are capable of retaining their long-term stable antigen binding activity in liquid formulations and in monkey or human blood (plasma), etc.

Moreover, with regard to the antibodies shown in (h) to (k) above, the hybridoma of Accession No. FERM BP-11337 was designated as "Mouse-Mouse hybridoma BA-1-3D" and deposited on February 1, 2011, the hybridoma of Accession No. FERM BP-10707 was designated as "Mouse-Mouse hybridoma: M3-1" and deposited on October 18, 2006, the hybridoma of Accession No. FERM BP-10899 was designated as "Mouse-Mouse hybridoma DI-2-14" and deposited on August 21, 2007, and the hybridoma of Accession No. FERM BP-10900 was designated as "Mouse-Mouse hybridoma DI-6" and deposited on August 21, 2007 with the International Patent Organism Depositary, the National Institute of Advanced Industrial Science and Technology in Japan (the name of the International Deposition Authority at the time of issue of their deposit receipts).

Anti-hDLK-1 antibodies available for use in the present invention preferably include those capable of competing with the above various anti-hDLK-1 antibodies, as exemplified by anti-hDLK-1 antibodies binding to sites (e.g., epitopes) to which the above various anti-hDLK-1 antibodies bind (recognize), etc.

### Epitope for anti-hDLK-1 antibody

The epitope (antigenic determinant) for the anti-hDLK-1 antibody is not limited in any way as long as it is at least a part of the antigen hDLK-1, but it is preferably, for example, at least a part of a region consisting of amino acids at positions 24 to 91 (i.e., a region comprising EGF-1 to EGF-2 of hDLK-1), a region consisting of amino acids at positions 92 to 167 (i.e., a region comprising EGF-3 to EGF-4 of hDLK-1), or a region consisting of amino acids at positions 131 to 244 (i.e., a region comprising EGF-4 to EGF-6 of hDLK-1) in the amino acid sequence of hDLK-1 shown in SEQ ID NO: 2. Above all, more preferred is a region comprising EGF-1 to EGF-2 of hDLK-1. The anti-hDLK-1 antibody recognizing such a region (binding to such a region), for example, has high internalization activity into tumor cells and is therefore very useful for use in immunoconjugates as described later.

### (iv) Genetically recombinant antibodies and antibody fragments

### Genetically recombinant antibodies

A preferred embodiment of the anti-hDLK-1 antibody may be a genetically recombinant antibody. Examples of a genetically recombinant antibody include, but are not limited to, a chimeric antibody and a humanized antibody, etc.

A chimeric antibody (i.e., a humanized chimeric antibody) is an antibody in which antibody variable regions of mouse origin are linked (conjugated) to constant regions of human origin (see, e.g., Proc. Natl. Acad. Sci. U.S.A. 81, 6851-6855 (1984)). For preparation of a chimeric antibody, gene recombination technology may be used for easy construction such that the thus linked antibody is obtained.

For preparation of a humanized antibody, complementarity determining regions (CDRs) from mouse antibody variable regions are grafted into human variable regions to prepare reconstituted variable regions whose framework regions (FRs) are of human origin and whose CDRs are of mouse origin (so-called CDR grafting). Then, these humanized reconstituted human variable regions are linked to human constant regions. For details of how to prepare such a humanized antibody, reference may be made to, for example, Nature, 321, 522-525 (1986); J. Mol. Biol., 196, 901-917 (1987); Queen C et al., Proc. Natl. Acad. Sci. USA, 86: 10029-10033 (1989); JP H04-502408 A (Japanese Patent No. 2828340; Queen et al.), etc. Further, the present invention may also encompass modified antibodies in which some amino acids (preferably one to several, more preferably one or two amino acids) in the H chain or L chain V region (except for CDR sequences) of the above humanized antibody are replaced with other amino acids. Such modified humanized anti-hDLK-1 antibodies may be humanized antibodies with much higher avidity (antigen binding activity), for example, such that they are capable of retaining their binding activity to cancer cells showing low antigen expression levels on the cell surface. In addition, these modified humanized anti-hDLK-1 antibodies may be capable of retaining their long-term stable antigen binding activity in liquid formulations and in monkey or human blood (plasma), etc.

The above chimeric and humanized antibodies are configured, for example, such that the N-glycoside-linked complex sugar chain in the antibody Fc region preferably has no fucose linked to N-acetylglucosamine at the reducing terminal of the sugar chain, as specifically exemplified by antibodies composed of genetically recombinant antibody molecules whose Fc region has a sugar chain in which the 1-position of the fucose is not α-liked to the 6-position of N-acetylglucosamine at the reducing terminal of the N-glycoside-linked complex sugar chain. Such an antibody allows a dramatic improvement in ADCC activity. It should be noted that this point (i.e., the characteristics of the N-glycoside-linked complex sugar chain in the antibody Fc region) is also preferred for the above polyclonal and monoclonal antibodies.

### Antibody fragments

In the present invention, a fragment of the anti-hDLK-1 antibody may also be used in combination with the anti-hDLK-1 antibody of the present invention or in place of the anti-hDLK-1 antibody of the present invention. Such an antibody fragment preferably has binding activity to hDLK-1, as in the case of the anti-hDLK-1 antibody of the present invention (including humanized antibody, etc., except for mouse antibody), and also preferred are those having *in vivo* antitumor activity or those recognizing the same epitope as the anti-hDLK-1 antibody of the present invention, but are not limited thereto.

Such an antibody fragment is intended to mean a partial region of the anti-hDLK-1 polyclonal antibody or the anti-hDLK-1 monoclonal antibody (i.e., an antibody fragment derived from the anti-hDLK-1 antibody of the present invention), and examples include Fab, Fab', F(ab')₂, Fv (variable fragment of antibody), single chain antibody (e.g., H chain, L chain, H chain V region, and L chain V region), scFv, diabody (scFv dimer), dsFv (disulfide stabilized V region), as well as peptides at least partially containing complementarity determining regions (CDRs), etc.

Fab is an antibody fragment having a molecular weight of about 50,000 and having antigen binding activity, which is composed of the N-terminal half of H chain and the full length of L chain linked via a disulfide bond, among fragments obtained by treating an antibody molecule with a protease, papain. Alternatively, Fab may also be prepared as follows: DNA encoding antibody Fab is inserted into a prokaryotic or eukaryotic expression vector, and this vector is introduced into a prokaryotic or eukaryotic organism for expression.

F(ab')₂ is an antibody fragment having a molecular weight of about 100,000 and having antigen binding activity, which is slightly larger than that composed of Fab fragments linked via disulfide bonds in the hinge region, among fragments obtained by treating an antibody molecule with a protease, pepsin. Alternatively, F(ab')₂ may also be prepared by linking Fab' fragments described later via thioether bonds or disulfide bonds.

Fab' is an antibody fragment having a molecular weight of about 50,000 and having antigen binding activity, which is obtained by cleaving the disulfide bonds in the hinge region of the above F(ab')₂. Alternatively, Fab' may also be prepared as follows: DNA encoding an antibody Fab' fragment is inserted into a prokaryotic or eukaryotic expression vector, and this vector is introduced into a prokaryotic or eukaryotic organism for expression.

scFv is an antibody fragment having antigen binding activity, which is composed of a single H chain V region (VH) and a single L chain V region (VL) linked via an appropriate peptide linker (P), i.e., a VH-P-VL or VL-P-VH polypeptide. For preparation of scFv, cDNAs encoding antibody VH and VL may be obtained to construct DNA encoding scFv, and this DNA may be inserted into a prokaryotic or eukaryotic expression vector, followed by introducing this expression vector into a prokaryotic or eukaryotic organism for expression.

Diabody is an antibody fragment composed of dimerized scFv fragments and having divalent antigen binding activity. The divalent antigen binding activity may be directed to the same antigen or to different antigens. For preparation of diabody, cDNAs encoding antibody VH and VL may be obtained to construct DNA encoding scFv such that the amino acid sequence of P has a length of 8 residues or less, and this DNA may be inserted into a prokaryotic or eukaryotic expression vector, followed by introducing this expression vector into a prokaryotic or eukaryotic organism for expression.

dsFv is an antibody fragment composed of VH and VL polypeptides, in each of which a single amino acid residue is replaced with a cysteine residue and which are linked via a disulfide bond between these cysteine residues. An amino acid residue to be replaced with a cysteine residue can be selected based on three-dimensional structure prediction of antibody according to the method reported by Reiter et al. (Protein Engineering, 7, 697-704, 1994). For preparation of dsFv, cDNAs encoding antibody VH and VL may be obtained to construct DNA encoding dsFv, and this DNA may be inserted into a prokaryotic or eukaryotic expression vector, followed by introducing this expression vector into a prokaryotic or eukaryotic organism for expression.

A CDR-containing peptide is configured to comprise at least one or more regions of VH or VL CDRs (CDRs 1 to 3). In the case of a peptide containing a plurality of CDRs, these CDRs may be linked directly or through an appropriate peptide linker. For preparation of a CDR-containing peptide, DNA encoding CDR in antibody VH or VL may be constructed, and the DNA may be inserted into a prokaryotic or eukaryotic expression vector, followed by introducing this expression vector into a prokaryotic or eukaryotic organism for expression. Alternatively, a CDR-containing peptide may also be prepared by chemical synthesis such as Fmoc (fluorenylmethyloxycarbonyl) and tBoc (t-butyloxycarbonyl) methods.

Antibody fragments for use in the present invention may be antibody fragments comprising a part or the whole of the antibody Fc region whose N-glycoside-linked complex sugar chain has no fucose linked to N-acetylglucosamine at the reducing terminal of the sugar chain, or alternatively, may be fusion proteins of the antibody fragments mentioned above with a part or the whole of the antibody Fc region whose N-glycoside-linked complex sugar chain has no fucose linked to N-acetylglucosamine at the reducing terminal of the sugar chain. Such antibody fragments allow a dramatic improvement in ADCC activity and are therefore preferred.

Specific examples of antibody fragments for use in the present invention include, but are not limited to, those comprising H chain V region CDRs 1 to 3 and L chain V region CDRs 1 to 3 in the various anti-hDLK-1 antibodies mentioned above, and those comprising the entire H chain V region and the entire the L chain V region in the various anti-hDLK-1 antibodies mentioned above.

### (v) Antibody-drug conjugate

The anti-hDLK-1 antibody and antibody fragments for use in the present invention may be in the form of conjugates with a compound having antitumor activity and/or cell killing activity. It should be noted that a product obtained when an antibody molecule or an antibody fragment molecule and a compound having antitumor activity and/or cell killing activity are each prepared in advance and then conjugated with each other is generally referred to as an immunoconjugate. Likewise, a product obtained when a protein toxin serving as a compound having antitumor activity and/or cell killing activity is ligated on its gene to a gene for an antibody or antibody fragment by gene recombination technology and allowed to be expressed as one protein (fusion protein) is generally referred to as an immunotoxin.

Examples of a compound having antitumor activity include doxorubicin, calicheamicin, mitomycin C, Auristatin E and so on. Examples of a compound having cell killing activity include saporin, ricin, pseudomonas exotoxin, diphtheria toxin and so on, with saporin and pseudomonas exotoxin being preferred for use.

Such a conjugate may be prepared in any manner, for example, by coupling an antibody to a drug via a disulfide bond or a hydrazone bond.

The anti-hDLK-1 antibody for use in the present invention is excellent in internalization activity into target tumor cells expressing hDLK-1. For this reason, when previously conjugated with a compound having antitumor activity and/or cell killing activity, the anti-hDLK-1 antibody allows such a compound to directly and highly selectively act on tumor cells. The thus obtained conjugate is very excellent in the ability of drug delivery to target tumor cells.

It should be noted that the internalization activity into cells may be evaluated as follows: an antibody is fluorescently labeled with rhodamine or the like and observed for its migratory behavior into cells and its localization therein under a fluorescence microscope, etc.

### (3) Pharmaceutical combination

The pharmaceutical combination of the present invention comprises lenvatinib or other form thereof and anti-hDLK-1 antibody or a fragment thereof as active ingredients.

The pharmaceutical combination of the present invention may be administered to a subject, i.e., a human or non-human mammal (e.g., mouse, rat, rabbit, sheep, pig, cow, cat, dog, monkey) by various routes of administration, as specifically exemplified by oral administration or parenteral administration (e.g., intravenous injection, intramuscular injection, intraperitoneal injection, subcutaneous injection, intrarectal administration, percutaneous administration).

Thus, the pharmaceutical combination of the present invention may be not only used alone, but also formulated with a pharmaceutically acceptable carrier into an appropriate dosage form in a manner commonly used, depending on the intended route of administration.

Dosage forms for oral formulations may be exemplified by tablets, powders, fine granules, granules, coated tablets, capsules, solutions for internal use, suspensions, emulsions, syrups and troches, etc., while dosage forms for parenteral formulations may be exemplified by injections (including drops), inhalants, ointments, nose drops, and liposomes, etc.

Examples of carriers which may be used to formulate these formulations include commonly used excipients, binders, disintegrants, lubricants, colorants, and correctives, as well as optionally stabilizers, emulsifiers, absorbefacients, surfactants, pH adjusters, antiseptics, antioxidants, extenders, humectants, surface active agents, dispersants, buffering agents, preservatives, solvent aids, and soothing agents, etc., which may be blended with known ingredients available for use as source materials for pharmaceutical formulations and then formulated in a standard manner.

Non-toxic ingredients available for this purpose may be exemplified by animal and vegetable oils such as soybean oil, beef tallow, and synthetic glycerides; hydrocarbons such as liquid paraffin, squalane, and hard paraffin; ester oils such as octyldodecyl myristate, and isopropyl myristate; higher alcohols such as cetostearyl alcohol, and behenyl alcohol; silicone resin; silicone oil; surfactants such as polyoxyethylene fatty acid esters, sorbitan fatty acid esters, glycerin fatty acid esters, polyoxyethylene sorbitan fatty acid esters, polyoxyethylene hydrogenated castor oil, and polyoxyethylene-polyoxypropylene block copolymers; water-soluble polymers such as hydroxyethylcellulose, polyacrylic acid, carboxyvinyl polymers, polyethylene glycol, polyvinylpyrrolidone, and methylcellulose; lower alcohols such as ethanol, and isopropanol; polyhydric alcohols (polyols) such as glycerin, propylene glycol, dipropylene glycol, sorbitol, and polyethylene glycol; sugars such as glucose, and sucrose; inorganic powders such as silicic anhydride, magnesium aluminum silicate, and aluminum silicate; inorganic salts such as sodium chloride, and sodium phosphate; and purified water. These ingredients may be in the form of salts or hydrates thereof.

Preferred examples of excipients include lactose, fructose, corn starch, sucrose, glucose, mannitol, sorbit, crystalline cellulose, and silicon dioxide, etc. Preferred examples of binders include polyvinyl alcohol, polyvinyl ether, methylcellulose, ethylcellulose, gum arabic, tragacanth, gelatin, shellac, hydroxypropylmethylcellulose, hydroxypropylcellulose, polyvinylpyrrolidone, polypropyleneglycol-polyoxyethylene block polymers, and meglumine, etc. Preferred examples of disintegrants include starch, agar, gelatin powder, crystalline cellulose, calcium carbonate, sodium bicarbonate, calcium citrate, dextrin, pectin, and carboxymethylcellulose calcium, etc. Preferred examples of lubricants include magnesium stearate, talc, polyethylene glycol, silica, and hydrogenated vegetable oils, etc. Preferred examples of colorants include those approved for addition to pharmaceutical products. Preferred examples of correctives include cocoa powder, menthol, aromatic powder, peppermint oil, borneol, and cinnamon powder, etc. These ingredients may be in the form of salts or hydrates thereof.

The dose of the pharmaceutical combination of the present invention may generally be determined extensively as appropriate for the age and body weight of a subject (patient) to be administered, the type and progression of disease, the route of administration, the frequency of administration (per day), the period of administration, etc., in consideration of the ratio of the active ingredients incorporated into the formulation. Moreover, in the pharmaceutical combination of the present invention, the active ingredients, i.e., lenvatinib or other form thereof and anti-hDLK-1 antibody or a fragment thereof may be administered substantially at the same time or administered sequentially in the order in which one is ahead of the other, without being limited thereto. In addition, these administrations are not limited in any way, and the amounts contained in the pharmaceutical combination may be administered at once or continuously.

A detailed explanation will be given below for the case where the pharmaceutical combination of the present invention is used as a parenteral formulation or an oral formulation.

For use as a parenteral formulation, the pharmaceutical combination of the present invention may usually be formulated into any dosage form. In the case of various types of injections, for example, they may be provided in the form of unit dose ampules or multi-dose containers or as freeze-dried powders which are dissolved again in a diluent before use. Such a parenteral formulation may comprise not only lenvatinib or other form thereof and anti-hDLK-1 antibody or a fragment thereof serving as active ingredients, but also various known excipients and/or additives as appropriate for each dosage form as long as the effect of the above active ingredients is not impaired. Examples of excipients and/or additives include water, glycerol, propylene glycol, and aliphatic polyalcohols such as polyethylene glycol, etc., in the case of various types of injections.

The dose (daily dose) of such a parenteral formulation is not limited in any way. For example, in the case of various types of injections, the dose may generally be set such that lenvatinib or other form thereof and anti-hDLK-1 antibody or a fragment thereof serving as active ingredients can be taken in an amount of 0.01 to 1000 mg, 0.05 to 500 mg or 0.1 to 50 mg, per kg body weight of a subject to be applied (e.g., a test subject, a patient), or alternatively, can be taken in an amount of 0.5 to 20 mg or can be taken in an amount of 1 to 10 mg.

For use as an oral formulation, the pharmaceutical combination of the present invention may usually be formulated into any dosage form among those mentioned above, or alternatively, may be formulated into a freeze-dried product which is dissolved again before use. Such an oral formulation may comprise not only lenvatinib or other form thereof and anti-hDLK-1 antibody or a fragment thereof serving as active ingredients, but also various known excipients and/or additives as appropriate for each dosage form as long as the effect of the above active ingredients is not impaired. Examples of excipients and/or additives include binders (e.g., syrup, gum arabic, gelatin, sorbitol, tragacanth, polyvinylpyrrolidone), fillers (e.g., lactose, sugar, corn starch, potato starch, calcium phosphate, sorbitol, glycine), lubricants (e.g., magnesium stearate, talc, polyethylene glycol, silica), disintegrants (e.g., various types of starches), and wetting agents (e.g., sodium lauryl sulfate), etc.

The dose (daily dose) of such an oral formulation may generally be set such that lenvatinib or other form thereof and anti-hDLK-1 antibody or a fragment thereof serving as active ingredients can be taken in an amount of 0.05 to 5000 mg, 0.1 to 1000 mg or 0.1 to 100 mg, per kg body weight of a subject to be applied (e.g., a test subject, a patient), or alternatively, can be taken in an amount of 0.5 to 50 mg or can be taken in an amount of 1 to 10 mg. Moreover, the ratio of the active ingredients incorporated into the oral formulation is not limited in any way and may be set as appropriate in consideration of the frequency of administration per day, etc.

The pharmaceutical combination of the present invention is capable of exerting a more potent and sustained antitumor effect than existing multikinase inhibitors in the treatment of hepatocellular carcinoma. For example, the pharmaceutical combination of the present invention allows suppression of cancer cell proliferation or allows tumor reduction or disappearance even after completing the administration of the pharmaceutical combination. The pharmaceutical combination of the present invention is very useful in terms of being capable of exerting an effect on patients with hepatocellular carcinoma who could not have been expected to experience any therapeutic effect.

### 3. Kit

The present invention can also provide a kit for treating hepatocellular carcinoma, which comprises lenvatinib or other form thereof and anti-hDLK-1 antibody or a fragment thereof as constituent elements.

In such a kit, lenvatinib or other form thereof and anti-hDLK-1 antibody or a fragment thereof may be provided, for example, in a dissolved state in consideration of their stability (storage quality) and easiness of use, etc.

Such a kit may also comprise other constituent elements, as appropriate, in addition to lenvatinib or other form thereof and anti-hDLK-1 antibody or a fragment thereof. For example, the kit may further comprise an antibody labeling substance, or alternatively, an immobilized reagent in which an antibody or a labeled product thereof is immobilized. The antibody labeling substance is intended to mean a substance labeled with an enzyme, a radioisotope, a fluorescent compound, a chemiluminescent compound or the like. Moreover, the kit may also comprise various buffers, sterilized water, various cell culture vessels, various reaction vessels (e.g., Eppendorf tubes), a blocking agent (e.g., bovine serum albumin (BSA), skimmed milk, goat serum or other serum components), a detergent, a surfactant, various plates, an antiseptic (e.g., sodium azide), an instruction manual for experimental operations (manufacturer's instructions) and so on.

The kit is required to comprise at least the above lenvatinib or other form thereof and the above anti-hDLK-1 antibody or fragment thereof as constituent elements. Thus, the kit may be configured to comprise the constituent elements essential for the treatment of hepatocellular carcinoma, either all together or separately from each other, without being limited thereto.

### EXAMPLES

The present invention will be further described in more detail by way of the following examples, although the present invention is not limited only to these examples.

### [Example 1]

Using a Hep3B xenograft model, lenvatinib mesylate and anti-hDLK-1 antibody were studied for their combined effect on tumorigenesis inhibitory activity.

The lenvatinib mesylate used here was commercially available (hereinafter simply referred to as "lenvatinib" in this example). The anti-hDLK-1 antibody used here was "HuBA-1-3D-1-A24G/T73K antibody" found in WO2014/054820 (hereinafter simply referred to as "HuBA-1-3D antibody" in this example), and DNA encoding this antibody protein was constructed in accordance with the procedures shown in WO2014/054820 (see the Example section in the specification), followed by production and preparation of this antibody protein in host cells using the GlymaxX^{®} technology (ProBioGen AG; see https://www.probiogen.de/genetic-glyco-engineering-adcc-glymaxx.html). It should be noted that the HuBA-1-3D-1-A24G/T73K antibody used here has an H chain V region consisting of the amino acid sequence shown in SEQ ID NO: 36 and an L chain V region consisting of the amino acid sequence shown in SEQ ID NO: 46.

1 × 10⁶ Hep3B cells were transplanted subcutaneously into the right flank of female NOD/ShiJic-scidJcl mice at 6 to 7 weeks of age (Day 0). At the stage where the mean tumor volume reached around 100 mm³ (Day 14), these mice were divided into the following groups: the control group (N = 8, 103.5 ± 18.1 mm³), the group receiving lenvatinib (3 mg/kg body weight) (N = 8, 102.8 ± 16.8 mm³), the group receiving HuBA-1-3D antibody (1 mg/kg body weight) (N = 8, 102.7 ± 15.0 mm³), and the group receiving both lenvatinib (3 mg/kg body weight) and HuBA-1-3D antibody (1 mg/kg body weight) (N = 8, 102.5 ± 12.5 mm³) (hereinafter referred to as the group receiving lenvatinib + HuBA-1-3D antibody). For a period of 12 days starting from Day 14, HuBA-1-3D antibody was administered twice a week (4 times in total; Days 14, 18, 22 and 25), and lenvatinib was administered 5 times a week (administration for 5 days and withdrawal for 2 days) (10 times in total; Days 14, 15, 16, 17, 18, 21, 22, 23, 24 and 25). The tumor volume was measured at a frequency of twice a week, and an animal whose tumor volume reached 1500 mm³ was excluded from observation.

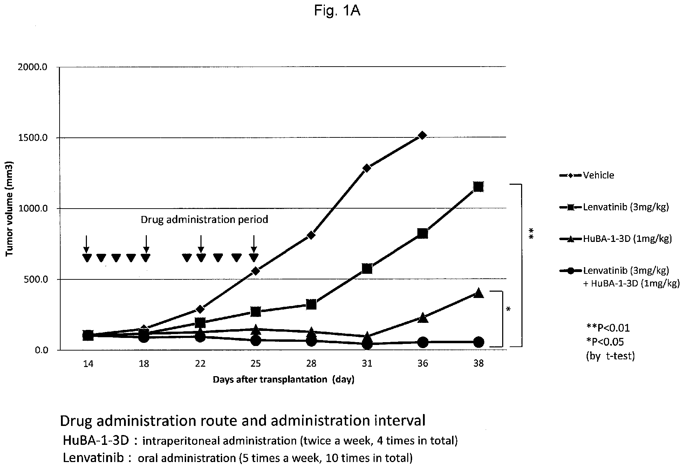

As a result, the tumor volume at 25 days after transplantation of the cancer cells (the final day of administration, Day 25) was 557.5 ± 240.5 mm³ in the control group, whereas it was 268.1 ± 175.7 mm³ in the group receiving lenvatinib, 144.6 ± 111.2 mm³ in the group receiving HuBA-1-3D antibody, and 66.2 ± 59.1 mm³ in the group receiving lenvatinib + HuBA-1-3D antibody. Thus, potent antitumor activity was confirmed in all the tested groups when compared to the control group. In addition, the efficacy after completion of administration was studied, indicating that at 31 days after transplantation of the cancer cells (6 days after completion of administration, Day 31), the tumor volume was 1282.8 ± 448.3 mm³ in the control group, 572.2 ± 456.0 mm³ in the group receiving lenvatinib, and 92.9 ± 79.3 mm³ in the group receiving HuBA-1-3D antibody, whereas the tumor volume was 40.3 ± 46.1 mm³ in the group receiving lenvatinib + HuBA-1-3D antibody. Further, at 38 days after transplantation of the cancer cells (13 days after completion of administration, Day 38), the tumor volume was 1148.5 ± 591.0 mm³ in the group receiving lenvatinib, and 402.3 ± 364.4 mm³ in the group receiving HuBA-1-3D antibody, whereas the tumor volume was 52.8 ± 63.9 mm³ in the group receiving lenvatinib + HuBA-1-3D antibody. Namely, in the group receiving lenvatinib + HuBA-1-3D antibody, potent antitumor activity was confirmed to be sustained even after completion of administration, when compared to the group receiving lenvatinib and the group receiving HuBA-1-3D antibody (Figure 1A).

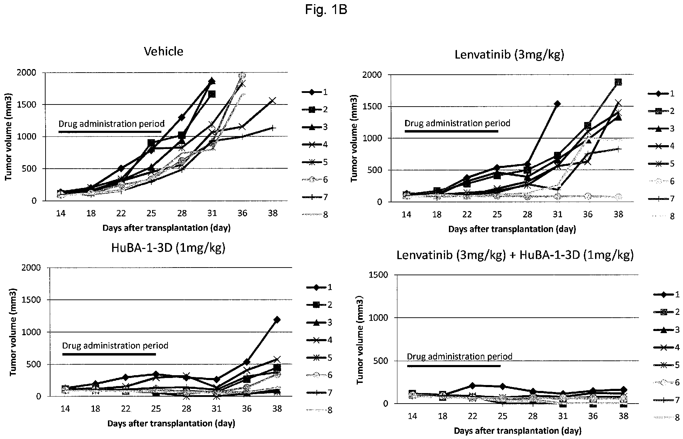

The time course of tumor volume in each animal is shown in Figure 1B. During the period of administration, tumor volume increase was suppressed in the group receiving lenvatinib and the group receiving HuBA-1-3D antibody when compared to the control group. However, after the period of administration, tumor volume increase was observed in almost all the animals in the group receiving lenvatinib and the group receiving HuBA-1-3D antibody. In contrast, in the group receiving lenvatinib + HuBA-1-3D antibody, tumor volume increase was suppressed in almost all the animals even after the period of administration.

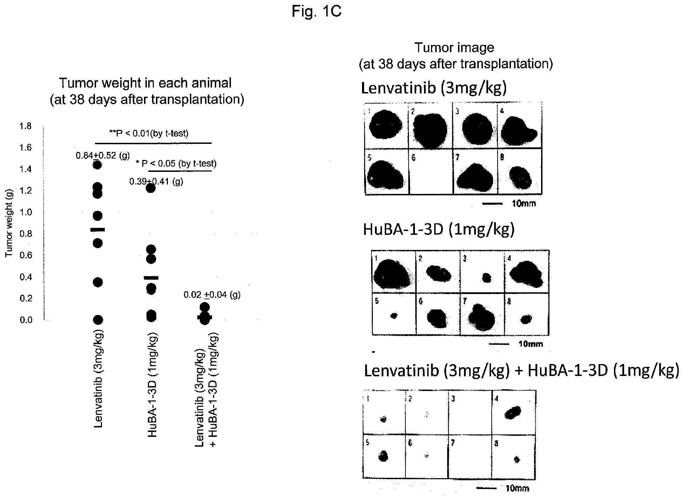

The weight and image of each tumor collected on the final day of observation (Day 38) are shown in Figure 1C. The tumor weight was 841.1 ± 515.8 mg in the group receiving lenvatinib (N = 7), 392.3 ± 412.9 mg in the group receiving HuBA-1-3D antibody (N = 8), and 22.9 ± 40.5 mg in the group receiving lenvatinib + HuBA-1-3D antibody (N = 8). Thus, the tumor weight was significantly smaller in the group receiving lenvatinib + HuBA-1-3D antibody than in the group receiving lenvatinib and the group receiving HuBA-1-3D antibody. In addition, when compared to the group receiving lenvatinib and the group receiving HuBA-1-3D antibody, the group receiving lenvatinib + HuBA-1-3D antibody had smaller tumors, and tumor disappearance was confirmed in half of the animals.

### [Example 2]

Using a HepG2 xenograft model, lenvatinib mesylate and anti-hDLK-1 antibody were studied for their combined effect on tumorigenesis inhibitory activity.

The lenvatinib mesylate used here was commercially available (hereinafter simply referred to as "lenvatinib" in this example). The anti-hDLK-1 antibody used here was "HuBA-1-3D-1-A24G/T73K antibody" found in WO2014/054820 (hereinafter simply referred to as "HuBA-1-3D antibody" in this example), and DNA encoding this antibody protein was constructed in accordance with the procedures shown in WO2014/054820 (see the Example section in the specification), followed by production and preparation of this antibody protein in host cells using the GlymaxX^{®} technology (ProBioGen AG; see https://www.probiogen.de/genetic-glyco-engineering-adcc-glymaxx.html). It should be noted that the HuBA-1-3D-1-A24G/T73K antibody used here has an H chain V region consisting of the amino acid sequence shown in SEQ ID NO: 36 and an L chain V region consisting of the amino acid sequence shown in SEQ ID NO: 46.

5 × 10⁶ HepG2 cells were transplanted subcutaneously into the right flank of female NOD/ShiJic-scidJcl mice at 7 weeks of age (Day 0). At the stage where the mean tumor volume reached around 100 mm³ (Day 10), these mice were divided into the following groups: the control group (N = 8, 118.4 ± 15.6 mm³), the group receiving lenvatinib (10 mg/kg body weight) (N = 8, 118.8 ± 15.2 mm³), the group receiving HuBA-1-3D antibody (1 mg/kg body weight) (N = 8, 119.8 ± 12.9 mm³), and the group receiving both lenvatinib (10 mg/kg body weight) and HuBA-1-3D antibody (1 mg/kg body weight) (N = 8, 119.7 ± 13.4 mm³) (hereinafter referred to as the group receiving lenvatinib + HuBA-1-3D antibody). For a period of 12 days starting from Day 10, HuBA-1-3D antibody was administered twice a week (4 times in total; Days 10, 13, 17 and 21), and lenvatinib was administered 5 times a week (administration for 5 days and withdrawal for 2 days) (10 times in total; Days 10, 11, 12, 13, 14, 17, 18, 19, 20 and 21). The tumor volume was measured at a frequency of twice a week, and an animal whose tumor volume reached 1500 mm³ was excluded from observation.

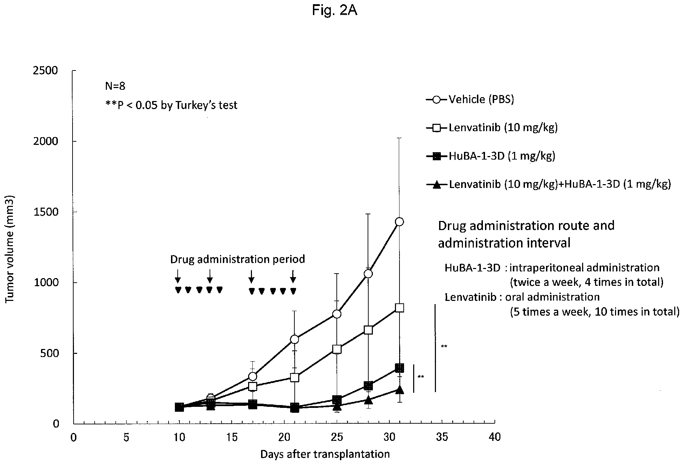

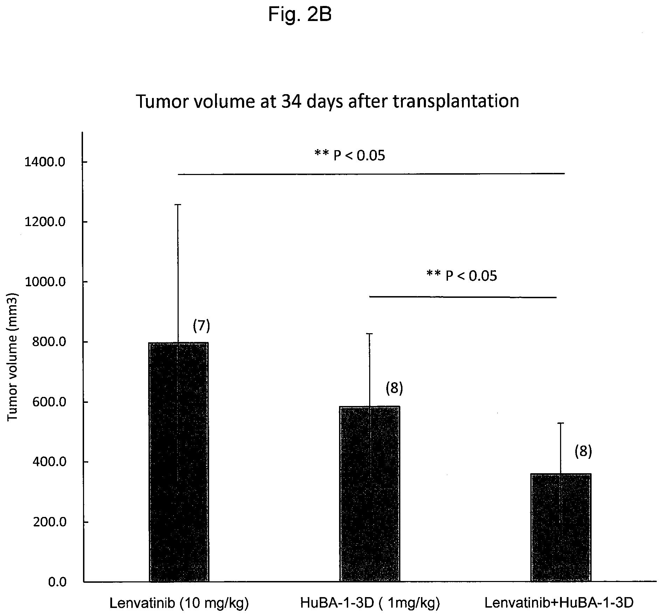

As a result, the tumor volume at 21 days after transplantation of the cancer cells (the final day of administration, Day 21) was 596.1 ± 201.6 mm³ in the control group (N = 8), whereas it was 326.2 ± 188.7 mm³ in the group receiving lenvatinib (10 mg/kg body weight) (N = 8), 116.4 ± 42.4 mm³ in the group receiving HuBA-1-3D (1 mg/kg) (N = 8), and 110.6 ± 33.3 mm³ in the group receiving lenvatinib (10 mg/kg body weight) + HuBA-1-3D (1 mg/kg) antibody (N = 8). Thus, potent antitumor activity was confirmed in all the tested groups when compared to the control group. Further, the efficacy after completion of administration was studied, indicating that at 31 days after transplantation of the cancer cells (10 days after completion of administration, Day 31), the tumor volume was 1427.7 ± 591.7 mm³ in the control group (N = 8), 817.4 ± 583.4 mm³ in the group receiving lenvatinib (10 mg/kg body weight) (N = 8), and 394.0 ± 169.8 mm³ in the group receiving HuBA-1-3D (1 mg/kg) antibody (N = 8), whereas the tumor volume was 240.8 ± 90.7 mm³ in the group receiving lenvatinib (10 mg/kg body weight) + HuBA-1-3D antibody (1 mg/kg) (N = 8). In the group receiving both lenvatinib (10 mg/kg body weight) and HuBA-1-3D (1 mg/kg), the tumor volume was significantly smaller when compared to the group receiving HuBA-1-3D (1 mg/kg) and the group receiving lenvatinib (10 mg/kg body weight), so that their combined administration was confirmed to enhance the antitumor effect (Figure 2A). At the time of Day 31, 4 of the 8 animals in the control group were sacrificed whose tumor volume exceeded 1500 mm³, while one of the 8 animals in the group receiving lenvatinib was sacrificed whose tumor volume exceeded 1500 mm³. The group receiving lenvatinib (10 mg/kg body weight) + HuBA-1-3D (1 mg/kg) in which the combined effect was observed at the time of Day 31, and the group receiving lenvatinib (10 mg/kg body weight) and the group receiving HuBA-1-3D (1 mg/kg) were observed until Day 34 (13 days after completion of administration), indicating that the tumor volume was 796.2 ± 461.7 mm³ in the group receiving lenvatinib (10 mg/kg body weight) (N = 7), and 584.1 ± 241.9 mm³ in the group receiving HuBA-1-3D (1 mg/kg) (N = 8), whereas the tumor volume was 358.0 ± 168.1 mm³ in the group receiving lenvatinib (10 mg/kg body weight) + HuBA-1-3D (1 mg/kg) (N = 8). Thus, in the group receiving both lenvatinib (10 mg/kg body weight) and HuBA-1-3D (1 mg/kg), the tumor volume was significantly smaller when compared to the group receiving HuBA-1-3D (1 mg/kg) and the group receiving lenvatinib (10 mg/kg body weight), so that their combined administration was confirmed to enhance the antitumor effect (Figure 2B).

### [Reference Example 1]

Two cell lines (Hep3B and HepG2) derived from human hepatocellular carcinoma were compared for their sensitivity to lenvatinib mesylate (commercially available; hereinafter simply referred to as "lenvatinib" in this example) in xenograft therapeutic models.

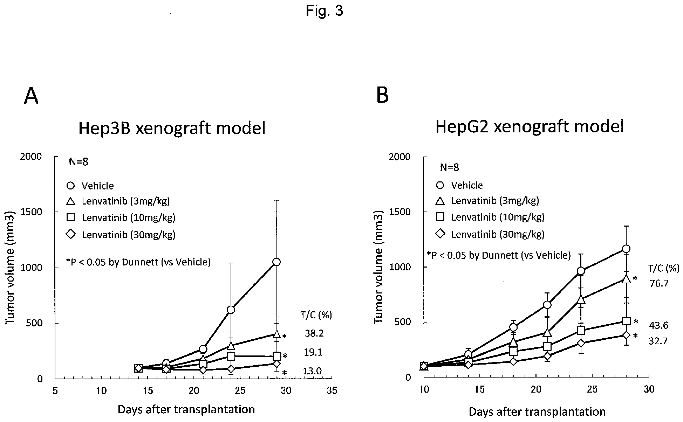

In a Hep3B xenograft model, NOD/SCID mice were transplanted with the cells subcutaneously, and then divided into groups of 8 mice each at the time when the mean tumor volume exceeded 100 mm³ (Day 14), and administration was started on the same day. The administration was conducted at a dose of 10 µL/g per mouse body weight via the oral route with an oral sonde, 5 times a week (administration for 5 days and withdrawal for 2 days) (12 times in total; Days 14, 15, 16, 17, 18, 21, 22, 23, 24, 25, 28 and 29) until the final day of the test. The tumor volume (mean ± standard deviation) at 29 days after transplantation (Day 29; the final day of the test) was 1050.3 ± 553.8 mm³ in the group receiving vehicle (water for injection) (N = 8), 400.9 ± 160.9 mm³ in the group receiving lenvatinib (3 mg/kg) (N = 8), 200.2 ± 133.3 mm³ in the group receiving lenvatinib (10 mg/kg) (N = 8), and 136.2 ± 33.8 mm³ in the group receiving lenvatinib (30 mg/kg) (N = 8). The tumor volume ratio (T/C) relative to the vehicle group on Day 29 was 38.2% (P<0.05), 19.1% (P<0.05) and 13.0% (P<0.05), respectively, in the groups receiving lenvatinib (3, 10 and 30 mg/kg) (Figure 3A).

On the other hand, in a HepG2 xenograft model, the sensitivity to lenvatinib was found to be lower than in the Hep3B model. NOD/SCID mice were transplanted with the cells subcutaneously, and then divided into groups of 8 mice each at the time when the mean tumor volume exceeded 100 mm³ (Day 10), and administration was started on the same day. The administration was conducted at a dose of 10 µL/g per mouse body weight via the oral route with an oral sonde, 5 times a week (administration for 5 days and withdrawal for 2 days) (14 times in total; Days 10, 11, 12, 13, 14, 17, 18, 19, 20, 21, 24, 25, 26 and 27) until the day before the final day of the test. The tumor volume (mean ± standard deviation) at 28 days after transplantation (Day 28; the final day of the test) was 1163.0 ± 205.1 mm³ in the group receiving vehicle (water for injection) (N = 8), 892.5 ± 220.7 mm³ in the group receiving lenvatinib (3 mg/kg) (N = 8), 506.8 ± 215.7 mm³ in the group receiving lenvatinib (10 mg/kg) (N = 8), and 380.0 ± 146.8 mm³ in the group receiving lenvatinib (30 mg/kg) (N = 8). The tumor volume ratio (T/C) relative to the vehicle group on Day 28 was 76.7% (P<0.05), 43.6% (P<0.05) and 32.7% (P<0.05), respectively, in the groups receiving lenvatinib (3, 10 and 30 mg/kg) (Figure 3B).

### INDUSTRIAL APPLICABILITY

The therapeutic agent and therapeutic method, etc., of the present invention are capable of exerting a more potent and sustained antitumor effect than existing multikinase inhibitors in the treatment of hepatocellular carcinoma, and are very useful, for example, in terms of being capable of exerting an effect on patients who could not have been expected to experience any therapeutic effect.

### Sequence Listing Free Text

SEQ ID NO: 23: recombinant DNA
SEQ ID NO: 24: synthetic construct (recombinant protein)
SEQ ID NO: 25: recombinant DNA
SEQ ID NO: 26: synthetic construct (recombinant protein)
SEQ ID NO: 27: recombinant DNA
SEQ ID NO: 28: synthetic construct (recombinant protein)
SEQ ID NO: 29: recombinant DNA
SEQ ID NO: 30: synthetic construct (recombinant protein)
SEQ ID NO: 31: recombinant DNA
SEQ ID NO: 32: synthetic construct (recombinant protein)
SEQ ID NO: 33: recombinant DNA
SEQ ID NO: 34: synthetic construct (recombinant protein)
SEQ ID NO: 35: recombinant DNA
SEQ ID NO: 36: synthetic construct (recombinant protein)
SEQ ID NO: 37: recombinant DNA
SEQ ID NO: 38: synthetic construct (recombinant protein)
SEQ ID NO: 39: recombinant DNA
SEQ ID NO: 40: synthetic construct (recombinant protein)
SEQ ID NO: 41: recombinant DNA
SEQ ID NO: 42: synthetic construct (recombinant protein)
SEQ ID NO: 43: recombinant DNA
SEQ ID NO: 44: synthetic construct (recombinant protein)
SEQ ID NO: 45: recombinant DNA
SEQ ID NO: 46: synthetic construct (recombinant protein)

## Claims

1. A pharmaceutical combination comprising
lenvatinib or a pharmacologically acceptable salt thereof; and
an antibody against human DLK-1, which has *in vivo* antitumor activity, or an antibody fragment derived from the antibody;
for use in a method of treating hepatocellular carcinoma;
wherein the antibody fragment has a binding activity to human DLK-1 and *in vivo* antitumor activity.

2. The pharmaceutical combination for use according to claim 1, wherein the tumor is hepatocellular carcinoma.

3. The pharmaceutical combination for use according to claim 1 or 2, wherein the antibody is a chimeric antibody or a humanized antibody.

4. The pharmaceutical combination for use according to any one of claims 1 to 3, wherein the antibody is at least one selected from the group consisting of:
(a) an antibody in which the amino acid sequences of H chain V region CDRs 1 to 3 are the amino acid sequences shown in SEQ ID NOs: 3 to 5, respectively, and the amino acid sequences of L chain V region CDRs 1 to 3 are the amino acid sequences shown in SEQ ID NOs: 6 to 8, respectively;
(b) an antibody in which the amino acid sequences of H chain V region CDRs 1 to 3 are the amino acid sequences shown in SEQ ID NOs: 9 to 11, respectively, and the amino acid sequences of L chain V region CDRs 1 to 3 are the amino acid sequences shown in SEQ ID NOs: 12 to 14, respectively;
(c) an antibody in which the amino acid sequence of the H chain V region consists of the amino acid sequence shown in SEQ ID NO: 16, and the amino acid sequence of the L chain V region consists of the amino acid sequence shown in SEQ ID NO: 18;
(d) an antibody in which the amino acid sequence of the H chain V region consists of the amino acid sequence shown in SEQ ID NO: 20, and the amino acid sequence of the L chain V region consists of the amino acid sequence shown in SEQ ID NO: 22;
(e) an antibody in which the amino acid sequence of the H chain V region consists of the amino acid sequence shown in SEQ ID NO: 24 or 26, and the amino acid sequence of the L chain V region consists of the amino acid sequence shown in SEQ ID NO: 28;
(f) an antibody in which the amino acid sequence of the H chain V region consists of the amino acid sequence shown in SEQ ID NO: 30, 32, 34 or 36, and the amino acid sequence of the L chain V region consists of the amino acid sequence shown in SEQ ID NO: 46;
(g) an antibody in which the amino acid sequence of the H chain V region consists of the amino acid sequence shown in SEQ ID NO: 38, 40, 42 or 44, and the amino acid sequence of the L chain V region consists of the amino acid sequence shown in SEQ ID NO: 46;
(h) an antibody produced by the hybridoma of Accession No. FERM BP-10899;
(i) an antibody produced by the hybridoma of Accession No. FERM BP-10707;
(j) an antibody produced by the hybridoma of Accession No. FERM BP-10900; and
(k) an antibody produced by the hybridoma of Accession No. FERM BP-11337.

5. The pharmaceutical combination for use according to any one of claims 1 to 4, wherein the antibody or antibody fragment is in the form of a conjugate with a compound having antitumor activity and/or cell killing activity.

6. The pharmaceutical combination for use according to any one of claims 1 to 5, which allows suppression of cancer cell proliferation or allows tumor reduction or disappearance even after completing the administration of the pharmaceutical combination.

7. An antibody against human DLK-1, which has *in vivo* antitumor activity, or an antibody fragment derived from the antibody, for use in a method of treating hepatocellular carcinoma in combination with lenvatinib or a pharmacologically acceptable salt thereof, wherein the antibody fragment has a binding activity to human DLK-1 and *in vivo* antitumor activity.

8. Lenvatinib or a pharmacologically acceptable salt thereof for use in a method of treating hepatocellular carcinoma in combination with an antibody against human DLK-1, which has *in vivo* antitumor activity, or an antibody fragment derived from the antibody, wherein the antibody fragment has a binding activity to human DLK-1 and *in vivo* antitumor activity.

9. A kit for treating hepatocellular carcinoma, which comprises:
lenvatinib or a pharmacologically acceptable salt thereof; and
an antibody against human DLK-1, which has *in vivo* antitumor activity, or an antibody fragment derived from the antibody;
wherein the antibody fragment has a binding activity to human DLK-1 and *in vivo* antitumor activity.

## Patentansprüche

1. Pharmazeutische Kombination, umfassend
Lenvatinib oder ein pharmakologisch annehmbares Salz davon; und
einen Antikörper gegen humanes DLK-1, der *in vivo* eine Antitumoraktivität aufweist, oder ein von dem Antikörper abgeleitetes Antikörperfragment;
zur Verwendung in einem Verfahren zur Behandlung von Leberzellkarzinomen;
wobei das Antikörperfragment eine Bindungsaktivität an humanes DLK-1 und *in vivo* eine Antitumoraktivität aufweist.

2. Pharmazeutische Kombination zur Verwendung nach Anspruch 1, wobei der Tumor ein Leberzellkarzinomen ist.

3. Pharmazeutische Kombination zur Verwendung nach Anspruch 1 oder 2, wobei der Antikörper ein chimärer Antikörper oder ein humanisierter Antikörper ist.

4. Pharmazeutische Kombination zur Verwendung nach einem der Ansprüche 1 bis 3, wobei der Antikörper mindestens einer ist, der ausgewählt ist aus der Gruppe bestehend aus:
(a) einem Antikörper, bei dem die Aminosäuresequenzen der CDRs 1 bis 3 der V-Region der H-Kette jeweils die in den SEQ ID NO: 3 bis 5 dargestellten Aminosäuresequenzen sind und die Aminosäuresequenzen der CDRs 1 bis 3 der V-Region der L-Kette jeweils die in den SEQ ID NO: 6 bis 8 dargestellten Aminosäuresequenzen sind;
(b) einem Antikörper, bei dem die Aminosäuresequenzen der CDRs 1 bis 3 der V-Region der H-Kette jeweils die in den SEQ ID NO: 9 bis 11 dargestellten Aminosäuresequenzen sind und die Aminosäuresequenzen der CDRs 1 bis 3 der V-Region der L-Kette jeweils die in den SEQ ID NO: 12 bis 14 dargestellten Aminosäuresequenzen sind;
(c) einem Antikörper, bei dem die Aminosäuresequenz der V-Region der H-Kette aus der in SEQ ID NO: 16 dargestellten Aminosäuresequenz besteht und die Aminosäuresequenz der V-Region der L-Kette aus der in SEQ ID NO: 18 dargestellten Aminosäuresequenz besteht;
(d) einem Antikörper, bei dem die Aminosäuresequenz der V-Region der H-Kette aus der in SEQ ID NO: 20 dargestellten Aminosäuresequenz besteht und die Aminosäuresequenz der V-Region der L-Kette aus der in SEQ ID NO: 22 dargestellten Aminosäuresequenz besteht;
(e) einem Antikörper, bei dem die Aminosäuresequenz der V-Region der H-Kette aus der in SEQ ID NO: 24 oder 26 dargestellten Aminosäuresequenz besteht und die Aminosäuresequenz der V-Region der L-Kette aus der in SEQ ID NO: 28 dargestellten Aminosäuresequenz besteht;
(f) einem Antikörper, bei dem die Aminosäuresequenz der V-Region der H-Kette aus der in SEQ ID NO: 30, 32, 34 oder 36 dargestellten Aminosäuresequenz besteht und die Aminosäuresequenz der V-Region der L-Kette aus der in SEQ ID NO: 46 dargestellten Aminosäuresequenz besteht;
(g) einem Antikörper, bei dem die Aminosäuresequenz der V-Region der H-Kette aus der in SEQ ID NO: 38, 40, 42 oder 44 dargestellten Aminosäuresequenz besteht und die Aminosäuresequenz der V-Region der L-Kette aus der in SEQ ID NO: 46 dargestellten Aminosäuresequenz besteht;
(h) einem Antikörper, der von dem Hybridom mit der Zugangsnummer FERM BP-10899 produziert wird;
(i) einem Antikörper, der von dem Hybridom mit der Zugangsnummer FERM BP-10707 produziert wird;
(j) einem Antikörper, der von dem Hybridom mit der Zugangsnummer FERM BP-10900 produziert wird;
und
(k) einem Antikörper, der von dem Hybridom mit der Zugangsnummer FERM BP-11337 produziert wird.

5. Pharmazeutische Kombination zur Verwendung nach einem der Ansprüche 1 bis 4, wobei der Antikörper oder das Antikörperfragment in Form eines Konjugats mit einer Verbindung vorliegt, die eine Antitumoraktivität und/oder zellabtötende Aktivität aufweist.

6. Pharmazeutische Kombination zur Verwendung nach einem der Ansprüche 1 bis 5, die eine Unterdrückung der Proliferation von Krebszellen oder eine Verkleinerung oder das Verschwinden des Tumors auch nach Beendigung der Verabreichung der pharmazeutischen Kombination ermöglicht.

7. Antikörper gegen humanes DLK-1, der *in vivo* eine Antitumoraktivität aufweist, oder ein von dem Antikörper abgeleitetes Antikörperfragment zur Verwendung in einem Verfahren zur Behandlung von Leberzellkarzinomen in Kombination mit Lenvatinib oder einem pharmakologisch annehmbaren Salz davon, wobei das Antikörperfragment eine Bindungsaktivität an humanes DLK-1 und *in vivo* eine Antitumoraktivität aufweist.

8. Lenvatinib oder ein pharmakologisch annehmbares Salz davon zur Verwendung in einem Verfahren zur Behandlung von Leberzellkarzinomen in Kombination mit einem Antikörper gegen humanes DLK-1, der *in vivo* eine Antitumoraktivität aufweist, oder einem von dem Antikörper abgeleiteten Antikörperfragment, wobei das Antikörperfragment eine Bindungsaktivität an humanes DLK-1 und *in vivo* eine Antitumoraktivität aufweist.

9. Kit zur Behandlung von Leberzellkarzinomen, umfassend:
Lenvatinib oder ein pharmakologisch annehmbares Salz davon; und
einen Antikörper gegen humanes DLK-1, der *in vivo* eine Antitumoraktivität aufweist, oder ein von dem Antikörper abgeleitetes Antikörperfragment;
wobei das Antikörperfragment eine Bindungsaktivität an humanes DLK-1 und *in vivo* eine Antitumoraktivität aufweist.

## Revendications

1. Combinaison pharmaceutique comprenant :
du lenvatinib ou un sel pharmacologiquement acceptable de celui-ci, et
un anticorps dirigé contre le DLK-1 humain, ayant une activité antitumorale *in vivo,* ou un fragment d'anticorps dérivé dudit anticorps ;
destinée à une utilisation dans une méthode de traitement du carcinome hépatocellulaire ;
ledit fragment d'anticorps ayant une activité de liaison au DLK-1 humain et une activité antitumorale *in vivo.*

2. Combinaison pharmaceutique destinée à une utilisation selon la revendication 1, ladite tumeur étant un carcinome hépatocellulaire.

3. Combinaison pharmaceutique destinée à une utilisation selon la revendication 1 ou 2, ledit anticorps étant un anticorps chimérique ou un anticorps humanisé.

4. Combinaison pharmaceutique destinée à une utilisation selon l'une quelconque des revendications 1 à 3, ledit anticorps étant au moins un anticorps sélectionné dans le groupe constitué de :
(a) un anticorps dans lequel les séquences d'acides aminés des CDR 1 à 3 de la région V de la chaîne H sont respectivement les séquences d'acides aminés représentées dans les SEQ ID NO : 3 à 5, et les séquences d'acides aminés des CDR 1 à 3 de la région V de la chaîne L sont respectivement les séquences d'acides aminés représentées dans les SEQ ID NO : 6 à 8 ;
(b) un anticorps dans lequel les séquences d'acides aminés des CDR 1 à 3 de la région V de la chaîne H sont respectivement les séquences d'acides aminés représentées dans les SEQ ID NO : 9 à 11, et les séquences d'acides aminés des CDR 1 à 3 de la région V de la chaîne L sont respectivement les séquences d'acides aminés représentées dans les SEQ ID NO : 12 à 14 ;
(c) un anticorps dans lequel la séquence d'acides aminés de la région V de la chaîne H est constituée de la séquence d'acides aminés représentée dans la SEQ ID NO : 16, et la séquence d'acides aminés de la région V de la chaîne L est constituée de la séquence d'acides aminés représentée dans la SEQ ID NO : 18 ;
(d) un anticorps dans lequel la séquence d'acides aminés de la région V de la chaîne H est constituée de la séquence d'acides aminés représentée dans la SEQ ID NO : 20, et la séquence d'acides aminés de la région V de la chaîne L est constituée de la séquence d'acides aminés représentée dans la SEQ ID NO : 22 ;
(e) un anticorps dans lequel la séquence d'acides aminés de la région V de la chaîne H est constituée de la séquence d'acides aminés représentée dans la SEQ ID NO : 24 ou 26, et la séquence d'acides aminés de la région V de la chaîne L est constituée de la séquence d'acides aminés représentée dans la SEQ ID NO : 28 ;
(f) un anticorps dans lequel la séquence d'acides aminés de la région V de la chaîne H est constituée de la séquence d'acides aminés représentée dans la SEQ ID NO : 30, 32, 34 ou 36, et la séquence d'acides aminés de la région V de la chaîne L est constituée de la séquence d'acides aminés représentée dans la SEQ ID NO : 46 ;
(g) un anticorps dans lequel la séquence d'acides aminés de la région V de la chaîne H est constituée de la séquence d'acides aminés représentée dans la SEQ ID NO : 38, 40, 42 ou 44, et la séquence d'acides aminés de la région V de la chaîne L est constituée de la séquence d'acides aminés représentée dans la SEQ ID NO : 46 ;
(h) un anticorps produit par l'hybridome correspondant au N° d'ordre FERM BP-10899 ;
(i) un anticorps produit par l'hybridome correspondant au N° d'ordre FERM BP-10707 ;
(j) un anticorps produit par l'hybridome correspondant au N° d'ordre FERM BP-10900 ;
(k) un anticorps produit par l'hybridome correspondant au N° d'ordre BP-11337.

5. Combinaison pharmaceutique destinée à une utilisation selon l'une quelconque des revendications 1 à 4, ledit anticorps ou fragment d'anticorps se présentant sous la forme d'un conjugué avec un composé ayant une activité antitumorale et/ou une activité de destruction cellulaire.

6. Combinaison pharmaceutique destinée à une utilisation selon l'une quelconque des revendications 1 à 5, qui permet la suppression de la prolifération des cellules cancéreuses ou permet la réduction ou la disparition de la tumeur même après l'achèvement de l'administration de la combinaison pharmaceutique.

7. Anticorps dirigé contre le DLK-1 humain, ayant une activité antitumorale *in vivo,* ou fragment d'anticorps dérivé dudit anticorps, destiné à une utilisation dans une méthode de traitement du carcinome hépatocellulaire en combinaison avec le lenvatinib ou un sel pharmacologiquement acceptable de celui-ci, ledit fragment d'anticorps présentant une activité de liaison au DLK-1 humain et une activité antitumorale *in vivo.*

8. Lenvatinib ou sel pharmacologiquement acceptable de celui-ci destiné à une utilisation dans une méthode de traitement du carcinome hépatocellulaire en combinaison avec un anticorps dirigé contre le DLK-1 humain, ayant une activité antitumorale *in vivo,* ou un fragment d'anticorps dérivé dudit anticorps, ledit fragment d'anticorps présentant une activité de liaison au DLK-1 humain et une activité antitumorale *in vivo.*

9. Trousse pour le traitement du carcinome hépatocellulaire, qui comprend :
du lenvatinib ou un sel pharmacologiquement acceptable de celui-ci ; et
un anticorps dirigé contre le DLK-1 humain, ayant une activité antitumorale *in vivo,* ou un fragment d'anticorps dérivé dudit anticorps ;
ledit fragment d'anticorps présentant une activité de liaison au DLK-1 humain et une activité antitumorale *in vivo.*
